# EUROPEAN PATENT APPLICATION

(11) **EP 2 426 499 A1**
(43) Date of publication of application: **07.03.2012**
(21) Application number: 10175242.6
(22) Date of filing: 03.09.2010
(51) Int. Cl.: G01N 33/68

(54) **Immunosuppressant monitoring by MALDI mass spectrometry**

(71) Applicant: Bruker Daltonik GmbH, 28359 Bremen (DE)
(72) Inventor: Urbani, Andrea, Professor, 00187 Rome (IT); Federici, Giorgio, Professor, 99162 Rome (IT)
(74) Representative: Kohler Schmid Möbus

(57) **Abstract**

The invention relates to therapeutic drug monitoring (TDM) by mass spectrometry, particularly to the monitoring of immunosuppressant levels in blood of patients with transplanted organs. The invention applies a fast liquid phase extraction procedure to reproducibly extract the therapeutic drug molecules from whole blood and mass spectrometric analysis on MALDI instruments, with a matrix substance for highest sensitivity and special sample deposition procedure for a reproducible ionization of the therapeutic drug molecules. Suitable internal standard substances added to the blood in exact amounts ensure a correct absolute quantification. The method is particularly suitable for immunosuppressants belonging to the class of macrocyclic lactones (sirolimus, tacrolimus, everolimus) and cyclic polypeptides (cyclosporin A), and even works as a multiplex method for all four immunosuppressants simultaneously.

## Description

### Field of invention

The invention relates to therapeutic drug monitoring (TDM) by mass spectrometry, particularly to the monitoring of immunosuppressant levels in blood of patients with transplanted organs.

### Prior Art

Pioneers of organ transplanting have become famous heroes known throughout the world. The biological and medical scientists investigating the immune system and developing immunosuppressants enabling organ transplanting, however, are widely unknown to the public. Such is life.

Monitoring therapeutic immunosuppressant levels in blood is generally important for all types of patients with immunosuppression, but particularly important for patients with transplants, because at least some of immunosuppressants have narrow therapeutical ranges, and it's easy to shift from a no activity condition with rejection of the transplanted organ to toxicity. Modern macrocyclic lactone immunosuppressants such as sirolimus, tacrolimus, everolimus or cyclic polypeptide immunosuppressants like cyclosporin A (also called "ciclosporin") are highly effective. However, they do have a remarkable narrow therapeutic range of 4-12 ng/ml (Sirolimus), 3-8 ng/ml (Everolimus) and 4-20 ng/ml (Tacrolimus) that causes a requirement for accurate monitoring of their levels in whole blood. In addition, the concentration in blood is not stably proportional to the amount of substance taken in orally; the proportionality varies even greatly with the condition of the patient. On the other hand, the quantification in whole blood is a complex and difficult procedure, given the very low doses routinely employed in transplanted patients.

Today, there are a variety of different monitoring methods for immunosuppressants in practical use, most of them being immunoassays. The term "immuno" in "immunoassays" refers to the use of antibodies which were originally generated by the immune system, not to the term "immuno" in "immunosuppression".

There are, for instance, the monitoring methods "ACMIA" = antibody conjugated magnetic immunoassay (Siemens Healthcare Diagnostics), "EMIT" = enzyme multiply immunoassay technique, "CEDIA" = cloned enzyme donor immunoassay (Microgenics GmbH), "FPIA"= fluorescent polarization immunoassay (Abbott), and "CMIA" = chemiluminescent microparticle immunoassay (Abbott). All these immunoassays are practically applied in different medical laboratories with roughly equal frequency. The original format of immunoassays, "RIA" = radioimmunoassay, has become rarely used today. Immunoassays are relatively expensive due to the costs of the specific antibodies.

Furthermore, there are two methods linked to HPLC = high performance liquid chromatography. Usual HPLC with simple UV detection is rarely applied in medical laboratories because it is extremely slow and not really specific with regard to immunosuppressant identification; but HPLC coupled with tandem mass spectrometry (HPLC-MS-MS), applying multiple reaction monitoring (MRM), is increasingly used. An example is "MassTrak" (Waters), using triple quadrupole mass spectrometers. MassTrak is based on an immunosuppressant specific kit comprising calibrators, quality controls, tuning mixture, internal standards, and special short HPLC columns. Up to now, this HPLC-MS-MS method is the single clinical assay using mass spectrometry. HPLC is used here to temporally separate substances from each other and from chemical background, but it increases the assay time. The substances separated by HPLC are ionized by electrospraying at atmospheric pressure; the ions are then transported into the vacuum system of the mass spectrometer. The first quadrupole mass filter of the triple quadrupole instrument selects immunosuppressant ions of the correct mass, the second quadrupole serves to fragment the ions in a distinct way, and the third quadrupole mass filter selects a characteristic fragment ion for detection. The method can be time programmed to detect other substances at different elution times, is highly sensitive but exhibits a relatively low throughput with a maximum of about 50 diagnostic runs per day.

As an alternative to the electrospray ionization in conjunction with HPLC-MS-MS method described above, there is another method to ionize organic molecules which cannot be thermally vaporized: MALDI = matrix assisted laser desorption and ionization. Samples for MALDI are prepared in solid form on mass spectrometric sample support plates, containing analyte molecules in extremely low amounts (around 10 amol - 100 fmol) within crystals of the MALDI matrix substances, usually aromatic acids of low molecular weight. The sample preparations are irradiated with pulsed UV light, generating plasma clouds in which some of the analyte molecules are ionized. The masses of these ions are determined by their flight time in corresponding MALDI time-of flight mass spectrometers (MALDI-TOF-MS).

Specialists in the field, however, will regard MALDI as the last method to be used for the monitoring of immunosuppressants, because (a) the mass range of the immunosuppressants (800 to 1200 Dalton) is overlapping with the strong chemical background of MALDI matrix clusters, and (b) MALDI is often reported as being highly non-quantitative. With MALDI, not only ions of the analyte molecules are formed, but also ions of the matrix substance, and ions of all the complexes of the matrix substances and their fragments generated in the hot laser plasma cloud. This chemical background reaches from low m/z values up to 1000 Dalton, overlapping with the mass spectrometric signals of tacrolimus, sirolimus and everolimus. In addition, the preparation long used for MALDI samples utilized drying droplets of a solution of matrix and analyte; thus, crystals were growing with irregular shape resulting in a very inhomogeneous distribution of crystals and of the analyte molecules inside the crystals. Upon laser irradiation, most parts of the sample preparation did not show any analyte ion signals, and others ("hot spots") delivered strong signals not reflecting the true concentration.

Only during the last years, preparation methods for thin layers of matrix material have been developed, which allow for quantitative work. Up to now, however, thin layer preparations of good quality have only been achieved for matrix materials which are not soluble in water. These can be loaded with aqueous analyte solutions without dissolving the matrix thin layer structure. The thin layer adsorbs the analyte molecules evenly on the surface of the micro crystallites. There are sample support plates commercially available with thin layer preparations of HCCA (= α-cyano-4-hydroxycinnamic acid), ideal for the ionization of peptides and proteins, but unfortunately this substance does not ionize the immunosuppressants with sufficiently high sensitivity. As a consequence, this method cannot be easily applied to immunosuppressants.

There are mainly four immunosuppressants currently used for transplant patients:

| Drug name | Monoisotopic Mol. Wt. | m/z [M+Na]⁺ | therapeutic window |
|---|---|---|---|
| Sirolimus | 913.55 Da | 936.54 | 4-12ng/ml |
| Tacrolimus | 803.48 Da | 826.47 | 4-20 ng/ml |
| Everolimus | 957.58 Da | 980.57 | 3-8ng/ml |
| Cyclosporin A | 1201.84 Da | 1224.83 | 75-150 ng/ml |

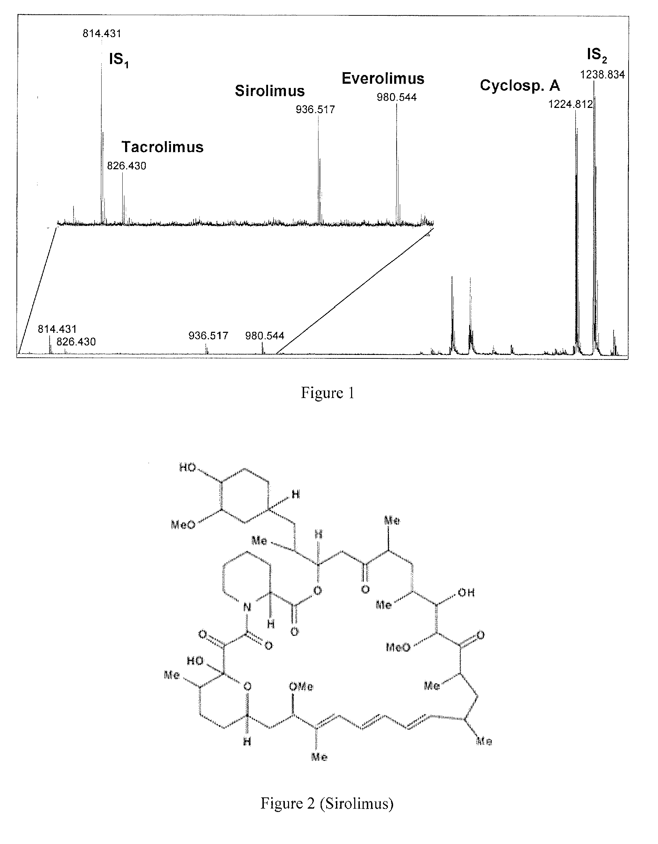

A typical case of therapeutic application is the monitoring of the immunosuppressant "sirolimus". Sirolimus is a macrocyclic lactone (a "macrolide") with a mechanism of action distinct from that of both cyclosporin A and tacrolimus. The structure is exhibited in Figure 2. Sirolimus decreases the acute rejection episodes in renal transplant patients. The optimum sirolimus concentration in blood amounts to 5 to 12 nanogram per milliliter. Increased levels of sirolimus above 15 nanogram per milliliter are associated with high toxicity, and generate thrombocytopenia, hyperlipidemia and high serum creatinine levels. Therefore frequent analyses of sirolimus in whole blood are required to avoid the rejection of the transplanted organ, when the concentration becomes too low, and to avoid collateral effects caused by too high concentrations.

All the methods in practical use are somewhat slow and as a rule very expensive. Regarding the increasing numbers of organ transplantations, there is still an urgent need for a highspeed low-cost method for the monitoring of immunosuppressants.

### Objective of the Invention

It is the objective of the invention to provide an inexpensive high-throughput method for the monitoring of therapeutic drugs, particularly for the monitoring of modern macrocyclic immunosuppressants.

### Summary of the Invention

The objective is achieved by mass spectrometric analysis
- with a fast, chromatography-free extraction procedure to reproducibly extract the therapeutic drug molecules from body fluids, particularly from whole blood,
- with a special preparation and deposition procedure for solid samples on a sample support plate in order to obtain reproducible ionization of the therapeutic drug molecules, and
- with a surface ionization method for the solid sample, particularly matrix-assisted laser desorption using a matrix substance for highest sensitivity.

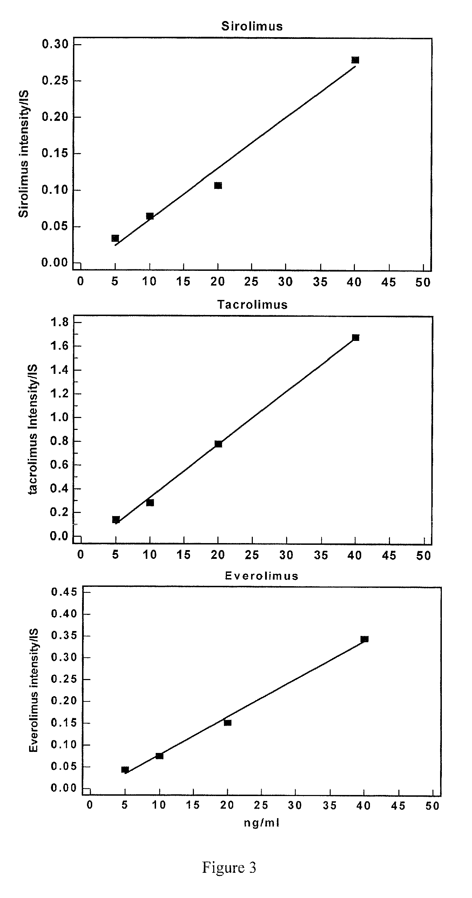

Suitable internal standard substances added to the body fluids in exact amounts ensure a correct absolute quantification. The method is particularly suitable for immunosuppressants belonging to the class of macrocyclic lactones (sirolimus, tacrolimus, everolimus) and cyclic polypeptides (cyclosporin A), and even works as a multiplex method for all four immunosuppressants simultaneously. The relatively narrow therapeutic range of the macrolides of approx. 3-20 ng/ml is well within the linear quantification range that the method can provide (Fig. 3).

Different extraction methods may be applied here, in principle, for the extraction of the drugs from the body fluid, including solid phase extraction, liquid/liquid extraction and affinity extraction. At present, best results were obtained by liquid phase extraction of the therapeutic drugs by emulsifying the aqueous body fluid in a vortexer with a hydrophobic organic solvent. Several organic solvents may be applied, most promising results for the immunosuppressant drugs were achieved with chlorobutane. Centrifugation helps to quickly separate the liquid phases. Evaporation of the organic phase within a clean container concentrates the drugs on the bottom wall, so that they can be resuspended in a small volume.

A microspotting procedure is used to generate spot areas with thin crystal layers on a ground steel sample support plate. A sample deposition method using DHB (= 2,5-dihydroxybenzoic acid) as matrix substance is achieving highest analytical sensitivity and lowest chemical background. On these thin layer spots, tiny volumes of DHB solution containing the resuspended analyte molecules are placed without completely dissolving the tiny crystals of the thin layers.

Surprisingly, the method provides quantitative results with a wide linear response of the analyte ion signals from a MALD1-TOF with sufficient accuracy (see Figure 3). The procedure offers high sensitivity and reproducibility without the use of chromatographic separation, thus allowing unprecedented short spectrum acquisition times of only a few seconds within the limit of the currently available UV lasers with 1 KHz repetition rate. Expensive antibodies are not required for the enrichment of the analytes. The new analysis procedure can be performed with surprisingly for the enrichment of the analytes. The new analysis procedure can be performed with surprisingly low reagents costs.

The tailored specificity of the extraction and crystallization protocol allows to obtain the necessary molecular discrimination for specific quantification without chromatographic separation in direct MS analysis without the use of MS/MS analyzers. The procedure allows for a multiplex quantification of all the immunosuppressants of the reported class without the need to change the preparation procedure. Thus the current method allows for direct monitoring of patients undergoing combined therapy with more then one single compound.

### Brief Description of the Figures

Figure 1 presents a mass spectrum in the charge-related mass range from *m*/*z* = 800 to 1200 Dalton, showing the mass signals (M+Na)⁺ of the therapeutic drugs sirolimus, tacrolimus, everolimus, and cyclosporin A, together with signals of the internal standards ascomycin and cyclosporin D.

Figure 2 depicts the primary structure of sirolimus.

Figure 3 shows measurement examples for the ranges of linear quantification for the three macrolides, reaching up to 50 nanogram per milliliter.

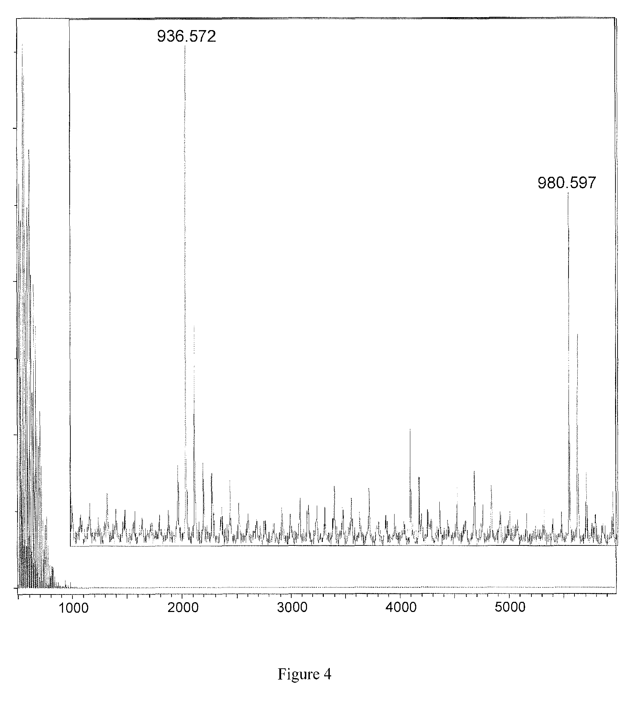

Figure 4 presents in the insert mass spectra of the ions (M+Na)⁺ of sirolimus and everolimus from true blood samples, spiked with 10 nanogram per milliliter each. The full mass spectrum shows the strong chemical background, decreasing from 500 Dalton to 1000 Dalton.

### Preferred Embodiments

A preferred embodiment according to the invention is a method for the monitoring of therapeutic drugs in human body fluids, wherein one or more internal standards are added to the body fluids in exactly defined amounts, the therapeutic drugs and the internal standard are separated from the body fluid by chromatography-free by liquid phase extraction, preparing solid samples from the extracted drugs on mass spectrometric sample support plates, and quantitatively analyzed by mass spectrometry by using ionization by matrix assisted laser desorption (MALDI). The internal standards should exhibit an extraction characteristic similar to those of the therapeutic drugs.

Good results were obtained by liquid phase extraction of the therapeutic drugs by emulsifying the aqueous body fluid in a vortexer with a hydrophobic organic solvent. Several organic solvents may be applied, most promising results for the immunosuppressant drugs were achieved with chlorobutane. Centrifugation helps to quickly separate the organic liquid phase from the aqueous liquid phase including all particles of the body fluid, e.g. blood particles. Evaporation of the supernatant organic phase in dry nitrogen within a clean container concentrates the drugs on the bottom wall, so that they can be resuspended in a small volume. Since no chromatographic separation methods need to be applied, the analysis procedure is extraordinarily fast.

The preferred method uses mass spectrometric ground steel sample support plates which are prespotted with microdroplets of a solution of 2,5-dihydroxibenzoic acid (DHB), to generate spot areas with thin layers of matrix material crystals on the plates. The therapeutic drugs, resuspended in DHB solution and desalted, can be pipetted onto these spots, dried, and measured in a MALDI mass spectrometer. A time-of-flight mass spectrometer with a reflector to achieve a high mass resolution is used.

Usually the monitoring of therapeutic drugs is performed on whole blood. Currently the most interesting application is the monitoring of immunosuppressants, particularly of macrocyclic immunosuppressants from the group sirolimus, tacrolimus, everolimus or cyclosporin A.

An example of an preparation protocol for the monitoring of these macrolides in whole blood may look like this:

An amount of 20 nanogram of an internal standard is added to 1 milliliter of whole blood sample, for example in form of 20 microliter of a solution of ascomycin (MW = 814.47) with a concentration of 1 nanogram per microliter. Furthermore, 1 milliliter of acetone and 2 milliliter of 4 % zinc sulfate in water are added and the mixture is vortexed for 30 seconds. The mixture is centrifuged for 10 minutes at 4000 revolutions per minute at room temperature to get rid of all blood particles. The supernatant is transferred to clean tubes containing 200 microlitres of 100 millimolar NaOH, then 2 milliliters of chlorobutane are added, and the mixture is vortexed for another 30 seconds to transfer the drugs into the organic phase. The mixture is again centrifuged at room temperature for 10 minutes at 4000 revolutions per minute, the organic phase is transferred to clean tubes and dried under pure nitrogen.

After drying, the drugs are resuspended in 10 microliters of a 0.1% solution of trifluoro acetic acid (TFA) in water mixed with 200 millimolar sodium acetate solution in a 4:1 proportion. The drug solution is then desalted with a commercial pipette tip that contains solid phase extraction media (e.g., ZipTip) and eluted again with 2 microliter of a 2:1 mixture of a solution of 3 milligram per milliliter super DHB matrix (sDHB) in acetonitrile (ACN) and 0.1 % TFA in water.

A ground steel sample support plate is prespotted with about 2 microliter per spot of a solution of 10 milligram super DHB dissolved in a milliliter of a mixture of 49,5 % acetonitrile, 49,5 % ethanol, and 1 % of 0.1% TFA in water. The microspotting process achieves spots of about 2 millimetres in diameter with thin layers of DHB. The 2 microliter elution liquid is placed on one of these spots, covering the spot completely and evenly, and dried.

The mass spectrometric sample support plate is inserted into the ion source of a MALDI time-of-flight mass spectrometer (MALDI-TOF-MS). Preferably, an instrument with an energy- focusing reflector is used; but in principle the monitoring should work with any MALDI mass spectrometer. Short UV laser light flashes with a few nanoseconds duration are focused onto the sample preparation, generating plasma clouds in which a part of the analyte molecules is ionized. The ions are accelerated from the ion source into the flight path of the spectrometer, and the flight times after which they reach an ion detector are measured accurately, resulting in a time-of-flight spectrum. The time-of-flight spectrum is transformed into a mass spectrum. Summing up a few hundred of such mass spectra improves the signal-to-noise ratio, allowing for precise concentration measurements of the therapeutic drugs in relation to the known concentration of the internal standards.

As internal standards for the monitoring of the macrocyclic immunosuppressants, the following substances have proved to work nicely:

| Internal standards: | Monoisotopic mass | [M+Na]⁺ |
|---|---|---|
| Ascomycin | 791.48 Da | 814.47 Da |
| Cyclosporin D | 1218.6 Da | 1238.85 Da |
| Cyclosporin B | 1187,8251 Da | 1209,8069 Da |

This preferred method can be varied in many ways. For example, other surface ionization techniques may be used here: laser desorption (LD), direct electrospray ionization (DESI), or others. Other matrix materials than DHB may be used, too, some of them may provide even higher sensitivities by higher ionization rates or by lower chemical background than DHB. Currently hundreds of matrix materials are known, some of them very specific for certain groups of analyte substances. Instead of ground steel plates prepared with matrix materials, other surfaces suitable for Laser desorption (LD) may be used. Even the use of simpler mass spectrometers (linear mode benchtop instruments) appears to be possible.

Different extraction methods may be applied for the extraction of the drugs from the body fluid, including solid phase extraction, liquid/liquid extraction and affinity extraction.

Instead of the internal standard materials described above, isotopically labeled immunosuppressants may be used, as they are expected to provide the best quantification accuracy and precision. Fourfold deuterated immunosuppressants or those with four ¹³C-atoms may be applied, for instance. However, their costs are significantly higher; they may be avoided if the increase in analysis quality is not required.

In total, the invention provides a low-cost high-throughput method for the monitoring of therapeutic drugs, particularly for modern immunosuppressants. The method can be at least partially automated.

To simplify the operation of the monitoring method according to this invention, solutions with internal standards, solutions with the different types of chemicals needed (as far as shipping is permitted), one-way-tools, suitable containers, and even prespotted mass spectrometer sample support plates may be prepackaged as kits. Such kits may be manufactured commercially.

The method provides quantitative results with a wide linear response of the analyte ion signals from the MALDI time-of-flight mass spectrometer with sufficient accuracy. The procedure offers high sensitivity and reproducibility without the use of chromatographic separation, thus allowing unprecedented short spectrum acquisition times of only a few seconds. Expensive antibodies are not required for the enrichment of the analytes, therefore, the new analysis procedure is associated with surprisingly low reagents costs.

## Claims

1. A method for the monitoring of therapeutic drugs in body fluid, comprising the steps:
(a) separating the therapeutic drugs from the body fluid by chromatography-free extraction, and
(b) quantitatively analyzing the therapeutic drugs by mass spectrometry.

2. The method according to Claim 1, wherein the body fluid is whole blood.

3. The method according to Claim 1 or 2, wherein liquid phase extraction, solid phase extraction or affinity extraction is used for the separating of the therapeutic drugs from the body fluid.

4. The method according to Claim 3, wherein liquid phase extraction with a hydrophobic organic solvent is used for the separating of the therapeutic drugs from the body fluid.

5. The method according to Claim 4, wherein the hydrophobic organic solvent is chlorobutane.

6. The method according to one of the Claims 1 to 5, wherein the therapeutic drugs are quantitatively analyzed by use of at least one internal standard with similar extraction characteristic, added to the body fluid prior to step (a).

7. The method according to Claim 6, wherein isotopically marked therapeutic drugs are used as internal standards.

8. The method according to Claim 6, wherein the therapeutic drugs are macrolide immunosuppressants from the group sirolimus, tacrolimus, everolimus or cyclosporine A, and wherein ascomycin, cyclosporin B or cyclosporin D or combinations thereof are used as internal standards.

9. The method according to one of the Claims 1 to 7, wherein the therapeutic drugs are immunosuppressants.

10. The method according to Claim 9, wherein the therapeutic drugs are immunosuppressants from the group sirolimus, tacrolimus, everolimus or cyclosporine A.

11. The method according to one of the Claims 1 to 10, wherein the quantitative analysis by mass spectrometry comprises a surface ionization method.

12. The method according to Claim 11, wherein matrix assisted laser desorption (MALDI) is used for surface ionization.

13. The method according to Claim 12, wherein an area for MALDI ionization is prepared by pre-spotting droplets of a solution with matrix substance to generate a thin layer of matrix substance crystals.

14. The method according to Claim 13, wherein the matrix substance is 2,5-dihydroxybenzoic acid (DHB).

15. The method according to Claim 13 or 14, wherein the therapeutic drugs are dissolved in a solution of the matrix substance and placed on the thin layer of matrix substance crystals.

16. A kit for performing one of the methods according to one of the Claims 1 to 15 comprising solutions of internal standards.

17. A kit according to Claim 16, additionally comprising weighed portions of matrix substance, solutions of chemicals, and/or prespotted sample support plates.

## Amended claims

### Amended claims in accordance with Rule 137(2) EPC.

**1.** A method for the monitoring of immunosuppressants belonging to the class of macrocyclic lactones and cyclic peptides in whole blood, comprising the steps:
(a) separating the immunosuppressants from the whole blood by chromatography-free liquid phase extraction using chlorobutane as hydrophobic organic solvent, and
(b) quantitatively analyzing the immunosuppressants by time-of-flight mass spectrometry using matrix assisted laser desorption/ionization (MALDI).

**2.** The method according to Claim 1, wherein the immunosuppressants are quantitatively analyzed by use of at least one internal standard with similar extraction characteristic, added to the whole blood prior to step (a).

**3.** The method according to Claim 2, wherein isotopically marked immunosuppressants are used as internal standards.

**4.** The method according to Claim 2, wherein the immunosuppressants are macrolide immunosuppressants from the group sirolimus, tacrolimus, everolimus or cyclosporine A, and wherein ascomycin, cyclosporin B or cyclosporin D or combinations thereof are used as internal standards.

**5.** The method according to Claim 1, wherein the immunosuppressants are from the group sirolimus, tacrolimus, everolimus or cyclosporine A.

**6.** The method according to one of the Claims 1 to 5, wherein an area for ionization by matrix assisted laser desorption/ionization (MALDI) is prepared by pre-spotting droplets of a solution with matrix substance to generate a thin layer of matrix substance crystals.

**7.** The method according to Claim 6, wherein the matrix substance is 2,5-dihydroxybenzoic acid (DHB).

**8.** The method according to Claim 6 or 7, wherein the immunosuppressants are dissolved in a solution of the matrix substance and placed on the thin layer of matrix substance crystals.

**9.** The method according to Claim 5, wherein the immunosupressantssirolimus, tacrolimus, everolimus and cyclosporine A are monitored in a multiplexed quantification.

**10.** The method according to one of Claims 1 to 9, wherein the immunosuppressants are quantitatively analyzed by time-of flight mass spectrometry using an energy-focusing reflector.
